# EUROPÄISCHE PATENTSCHRIFT

(11) **EP 1 986 926 B1**
(45) Veröffentlichungstag und Bekanntmachung des Hinweises auf die Patenterteilung: **27.10.2010**
(21) Anmeldenummer: 07703314.0
(22) Anmeldetag: 07.02.2007
(51) Int. Cl.: B65D 51/00, A61J 1/00, A61M 5/24, A61M 5/32

(54) **SPRITZE**
SYRINGE
SERINGUE

(30) Priorität: 14.02.2006 DE 102006006672
(43) Veröffentlichungstag der Anmeldung: 05.11.2008
(73) Patentinhaber: Arzneimittel GmbH Apotheker Vetter & Co. Ravensburg, 88212 Ravensburg (DE)
(72) Erfinder: GLOCKER, Joachim, 88250 Weingarten (DE)
(74) Vertreter: Gleiss, Alf-Olav
(86) Internationale Anmeldenummer: PCT/EP2007/001017
(87) Internationale Veröffentlichungsnummer: WO 2007/093307

(56) Entgegenhaltungen:
- EP-A1- 0 328 504
- EP-A1- 0 832 822
- EP-A1- 0 917 882
- WO-A-88/00478

## Beschreibung

Die Erfindung betrifft eine Spritze oder Karpule mit einer Membran und einer Abdeckeinrichtung gemäß Oberbegriff des Anspruchs 1.

Spritzen oder Karpulen der hier angesprochenen Art sind bekannt. Sie weisen einen Zylinder auf, der einen Hohlraum umschließt, der zur Aufnahme der Spritzenflüssigkeit dient, und ein Ende, das einen Zugang zum Hohlraum aufweist. Um die Flüssigkeit im Hohlraum des Zylinders steril zu halten, schließt eine Membran den Zugang dicht ab. Diese Membran ist derart ausgestaltet, dass sie einen Einstichbereich aufweist, der bei Anwendung der Spritze oder Karpule von einer Nadel durchstechbar ist.

Es hat sich herausgestellt, dass sich bei der Lagerung solcher Spritzen oder Karpulen Schmutzpartikel auf der von dem Zylinderende abgewandten Oberfläche der Membran ablagern können. Diese können bei Einstich einer Nadel durch die Membran in die Spritzenflüssigkeit gelangen und diese verunreinigen. Um dieses Problem zu lösen, wird das Ende des Spritzenzylinders mit einer Abdeckeinrichtung versehen, die den Nadeleinstichbereich der Nadel steril abdeckt und die einen den Nadeleinstichbereich überspannenden Dichtbereich umfasst. Mit dieser Abdeckeinrichtung wird die Membran vor Kontamination während der Lagerung geschützt. Des Weiteren weist das Ende des Zylinders eine die Membran überspannende Kappe auf, die eine Sollbruchlinie aufweist und am Zylinder gehalten wird. Will man die Kappe vor dem Einstichvorgang vom Zylinder lösen, so lässt sich diese oder ein Teil davon einfach über die Sollbruchlinie abtrennen. In EP 0 917 882 B1 wird eine Sicherheitskappe beschrieben, die eine Membran überdeckt, die über eine Kappe am Ende des Zylinders befestigt ist. Diese Kappe ist zweiteilig ausgebildet, wobei ein erster Teilbereich den Einstichbereich der Membran überdeckt und ein zweiter Teilbereich am Zylinder befestigt ist. Beide Teilbereiche sind über eine Sollbruchlinie verbunden. Die Kappe drückt die Abdeckeinrichtung gegen den Einstichbereich. Nachteilig ist, dass dadurch im Bereich der Sollbruchlinie axiale Spannungen auftreten, die bei der Lagerung der Spritze dazu führen können, dass die Sollbruchlinie aufplatzt, sich dann der erste Teilbereich vom zweiten Teilbereich löst und der Einstichbereich der Membran freigelegt wird.

Aufgabe der Erfindung ist es daher, eine Spritze oder Karpule zu schaffen, bei der die Membran steril abgedeckt wird und an der Sollbruchlinie der Kappe keine axialen Spannungen erzeugt werden.

Zur Lösung dieser Aufgabe wird eine Spritze oder Karpule vorgeschlagen, die die in Anspruch 1 genannten Merkmale aufweist. Sie zeichnet sich durch eine Abdeckeinrichtung aus, deren Dichtbereich unabhängig von im Bereich der Sollbruchlinie aufzubringenden Axialkräften den Nadeleinstichbereich steril abdeckt.

Bevorzugt wird ein Ausführungsbeispiel der Spritze oder Karpule, das sich dadurch auszeichnet, dass der Dichtbereich eine den Nadeleinstichbereich überspannende Siegelfolie umfasst. Durch diese Ausgestaltung wird sichergestellt, dass die Membran steril abgedeckt wird und keine Verunreinigungen in den Einstichbereich gelangen können.

Besonders bevorzugt wird ein Ausführungsbeispiel, das sich dadurch auszeichnet, dass die Abdeckeinrichtung einen Vorsprung und eine den Vorsprung zumindest bereichsweise aufnehmende Ausnehmung aufweist, und dass der Außendurchmesser des Vorsprungs so auf den Innendurchmesser der Ausnehmung abgestimmt ist, dass der Dichtbereich durch eine radiale Dichtung gebildet wird. Da die Dichtung radial realisiert ist, können keine Axialkräfte auftreten, die die Sollbruchlinie während einer Lagerung der Spritze aufsprengen können.

Weitere Ausgestaltungen ergeben sich aus den Unteransprüchen.

Die Erfindung wird im Folgenden anhand der Zeichnung näher erläutert. Es zeigen:
- Figur 1: einen Längsschnitt eines Endes eines Spritzenzylinders ohne Abdeckeinrichtung;
- Figur 2: eine perspektivische Darstellung eines Endes eines Zy- linders, der mit einer Siegelfolie abgedeckt ist;
- Figur 3: einen Längsschnitt eines Endes eines ersten Ausfüh- rungsbeispiels eines Spritzenzylinders mit einer Kappe und einer Abdeckeinrichtung;
- Figur 4: einen Längsschnitt eines Endes eines zweiten Ausfüh- rungsbeispiels eines Zylinders mit einer Abdeckeinrich- tung und
- Figur 5: einen Längsschnitt eines Endes eines dritten Ausfüh- rungsbeispiels eines Zylinders.

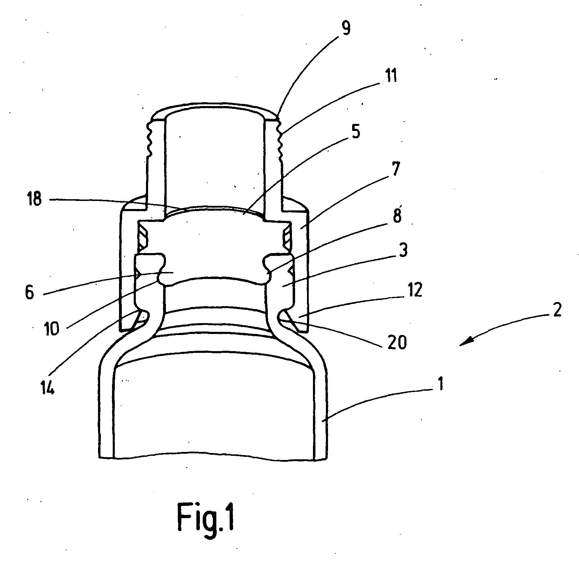

Figur 1 zeigt einen Längsschnitt durch einen Zylinder 1 einer Spritze 2 oder Karpule. Auf einem Ende 3 des Zylinders 1 liegt eine Membran 5 auf. Diese schließt den Zylinder 1 dicht ab. Des Weiteren weist die Membran 5 auf der dem Zylinder 1 zugewandten Seite einen Vorsprung 6 auf, der in das Innere des Zylinders 1 eingreift. Auf der Oberfläche des Vorsprungs 6 ist eine radial hervorstehende Wulst 8 erkennbar, die in eine Ausnehmung 10 in der Innenseite des Endes 3 eingreift. Dadurch wird die Membran 5 am Ende 3 des Zylinders 1 gehalten. Zusätzlich ist zum Fixieren der Membran 5 auf das Ende 3 ein Verschlusselement 7 aufgesetzt. Dieses umgreift die Membran 5 und das Ende 3 des Zylinders 1 und weist an seinem dem Zylinder 1 zugewandten Ende 12 mindestens einen Haken 14 auf. Dieser greift in eine Ausnehmung 20 ein, die sich zwischen dem Ende 3 des Zylinders 1 und dem Zylinder 1 selbst befindet. Damit ist das Verschlusselement 7 am Ende 3 des Zylinders 1 befestigt. Die Membran 5 befindet sich zwischen dem Verschlusselement 7 und dem Ende 3. Zusätzlich weist das Verschlusselement 7 einen Ansatz 9 auf. Dieser ist hohl, zylinderförmig ausgebildet und ist mit einem Außengewinde 11 versehen. Das Verschlusselement 7 bedeckt aufgrund des hohl ausgebildeten Ansatzes 9 die Membran 5 nicht vollständig. Der Bereich der Membran 5, der nicht vom Verschlusselement 7 bedeckt ist und von einer Nadel bei Anwendung der Spritze durchstochen werden kann, wird Einstichbereich 18 oder Nadeleinstichbereich genannt.

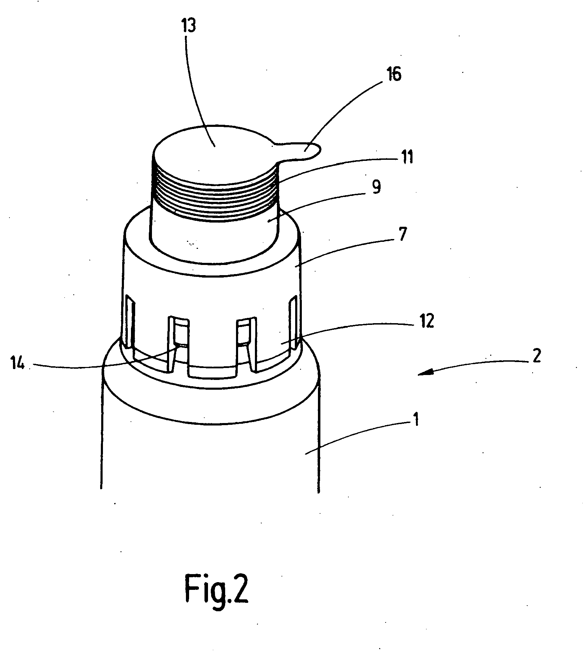

Figur 2 zeigt eine erste Ausführungsform der Spritze 2 mit einer Abdeckeinrichtung. Gleiche Teile sind mit gleichen Bezugsziffern versehen, sodass insofern auf die Beschreibung von Figur 1 verwiesen wird. Die perspektivische Darstellung zeigt, dass die Abdeckeinrichtung in diesem Ausführungsbeispiel als auf dem Ansatz 9 angebrachte Siegelfolie 13 ausgebildet ist. Diese umfasst einen den Nadeleinstichbereich überspannenden Dichtbereich.

Die Spritzenflüssigkeit im Zylinder 1 wird durch die in dieser Figur nicht sichtbare Membran 5 dicht eingeschlossen. Die Membran 5 wird durch das Verschlusselement 7 auf dem Ende des Zylinders 1 gehalten. Damit keine Verunreinigungen in den noch freiliegenden Bereich der Membran 5, also den Einstichbereich, gelangen können, wird dieser Einstichbereich durch die Siegelfolie 13 überspannt. Die Siegelfolie 13 wird am Verschlusselement 7 des Zylinders 1 mittels einer Klebeverbindung befestigt. Die Siegelfolie 13, die auf die vom Zylinder abgewandten Seite des Ansatzes 9 geklebt wird, kann Kunststoff und/oder Aluminium umfassen oder aus diesen Materialien bestehen und weist als Abziehhilfe an einer Stelle des Randes vorzugsweise mindestens eine Abziehlasche 16 auf. Damit kann bei Verwendung der Spritze 2 die Siegelfolie 13 problemlos abgezogen werden. Danach kann eine Nadel durch den Einstichbereich 18 in den Zylinder 1 eingeführt werden. Wesentlich ist, dass bei dieser Ausführungsform bei Verwendung einer zweiteiligen, die Membran überspannenden Kappe keine Axialkräfte auf eine Sollbruchlinie der Kappe wirken.

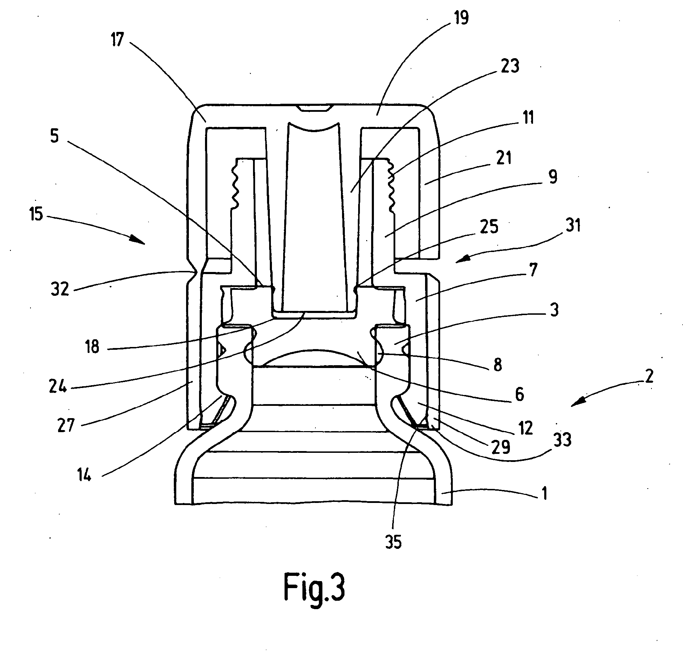

Figur 3 zeigt eine weitere Ausführungsform. Gleiche Teile sind mit gleichen Bezugsziffern versehen, sodass insofern auf die Beschreibung der vorigen Figuren verwiesen wird. Figur 3 zeigt das Ende 3 des Zylinders 1, die Membran 5 und das Verschlusselement 7, außerdem eine die Membran 5 und das Verschlusselement 7 überspannende zweiteilige Kappe 15. Diese weist einen ersten Teilbereich 17 auf, der einen Boden 19, einen von diesem ausgehenden, zentrischen Mantel 21 und einen von der Innenseite des Bodens 19 entspringenden zentrierten Zapfen 23 aufweist. Des Weiteren weist die Membran 5 eine Ausnehmung 24 auf, wobei der Zapfen 23 des ersten Teilbereichs 17 der Kappe 15 durch den hohl ausgebildeten Ansatz 9 des Verschlusselements 7 in die Ausnehmung 24 der Membran 5 eingreift und damit einen Vorsprung bildet. Der Einstichbereich 18 wird durch den Boden der Ausnehmung 25 der Membran 5 gebildet. Der Außendurchmesser des Zapfens 23 und der Innendurchmesser der Ausnehmung 24 sind so aufeinander abgestimmt, dass die Außenwand des Zapfens 23 dichtend an der Innenwand der Ausnehmung 24 der Membran 5 anliegt.

Durch diese Anordnung werden also durch die Kappe 15 eine Abdeckeinrichtung und durch den Zapfen 23 und die Ausnehmung 24 ein Dichtbereich ausgebildet, die einen Vorsprung und eine den Vorsprung aufnehmende Ausnehmung aufweisen.

Die Dichtwirkung im Dichtbereich kann dadurch noch verbessert werden, dass auf der Außenseite des hier als Zapfen 23 realisierten Vorsprungs und/oder auf der Innenseite der den Vorsprung aufnehmenden Ausnehmung 24 mindestens eine Wulst vorgesehen ist. Bei dem in Figur 3 dargestellten Ausführungsbeispiel ist auf der Innenseite der Ausnehmung 24 der Membran 5 eine Wulst 25 vorgesehen, die radial nach innen in Richtung auf eine gedachte Mittelachse der Ausnehmung 24 vorsteht.

Ein zweiter Teilbereich 27 der zweiteiligen Kappe 15 ist über eine Sollbruchlinie 31 mit dem ersten Teilbereich 17 der Kappe 15 verbunden. Die Sollbruchlinie kann durch einen dünnwandigen Bereich realisiert werden. Vorzugsweise ist vorgesehen, dass die beiden Teilbereiche 17 und 27 der Kappe 15 durch Abreißstege miteinander verbunden sind. In Figur 3 ist links ein derartiger Abreißsteg 32 der Sollbruchlinie 31 ersichtlich. Die Anzahl der Abreißstege 32 kann an die Festigkeit des Materials der Kappe 15 und an die bei Transport und Lagerung der Spritze 2 auf die Kappe 15 wirkenden Kräfte angepasst werden.

Der zweite Teilbereich 27 kann zur Fixierung am Ende 3 beispielsweise über eine Klebeverbindung mit dem Verschlusselement 7 verbunden sein. Das vom ersten Teilbereich 17 abgewandte Ende 29 des zweiten Teilbereichs 27 der Kappe 15 weißt - wie hier dargestellt - vorzugsweise mindestens einen keilförmigen Vorsprung 33 auf, der in eine Vertiefung 35 an dem dem Zylinder 1 zugewandten Ende 12 des Verschlusselements 7 eingreift und damit der Kopplung des zweiten Teilbereichs 27 an das Verschlusselement 7 dient.

Wesentlich ist, dass an der Sollbruchlinie 31 keine in axialer Richtung - also in Richtung einer gedachten Mittelachse der Kappe - wirkenden Kräfte auftreten. Dadurch wird sichergestellt, dass bei der Lagerung der Spritze 2 nicht ungewollt Abreißstege 32 aufgesprengt werden. Damit wird verhindert, dass der erste Teilbereich 17 vom zweiten Teilbereich 27 getrennt wird und der Einstichbereich 18 der Membran 5 frei liegt. Die axialen Kräfte werden dadurch vermieden, dass durch die Wulst 25 der Membran 5 eine radiale Dichtung erzeugt wird, indem die Wulst 25 radial an die Außenfläche des Zapfens 23 des ersten Teilbereichs 17 der Kappe 15 angedrückt wird.

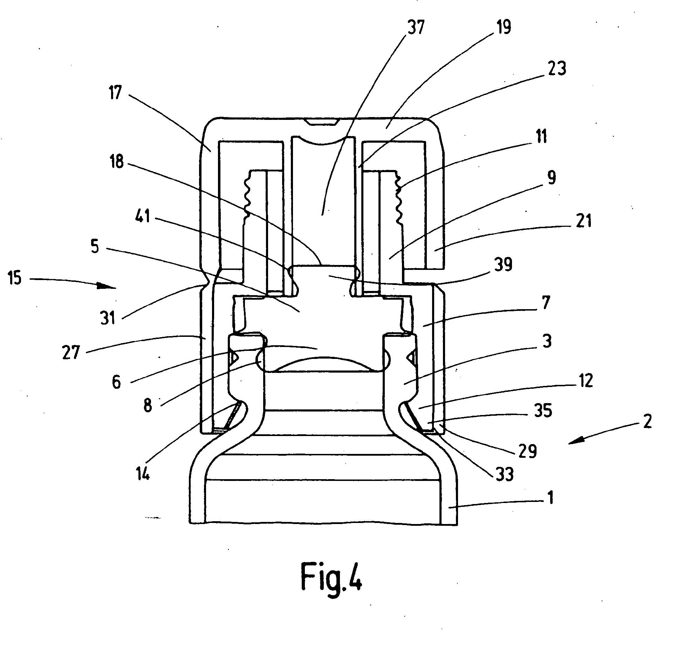

Figur 4 zeigt ein weiteres Ausführungsbeispiel der Spritze 2. Gleiche Teile werden mit gleichen Bezugsziffern versehen, sodass auf eine wiederholte Beschreibung verzichtet wird. Bei dieser Ausführungsform weisen der Zapfen 23 eine Ausnehmung 37 und die Membran 5 einen Vorsprung 39 auf. Der Einstichbereich 18 befindet sich auf der Oberseite des Vorsprungs 39 der Membran 5. Der Außendurchmesser des Vorsprungs 39 ist so auf den Innendurchmesser der Ausnehmung 37 des Zapfens 23 des ersten Teilbereichs 17 der zweiteiligen Kappe 15 abgestimmt, dass im Berührungsbereich ein Dichtbereich mit einer radialen Dichtung realisiert wird. Der Vorsprung 39 der Membran 5 weist vorzugsweise mindestens eine radial von der Außenfläche des Vorsprungs 39 vorstehende umlaufende Wulst 41 auf. Damit wird zusätzlich eine radiale Druckkraft aufgebaut, die die radiale Dichtung verbessert. Durch die radiale Dichtung herrschen somit keine axialen Kräfte an der Sollbruchlinie 31, sodass diese nicht aufgesprengt werden kann.

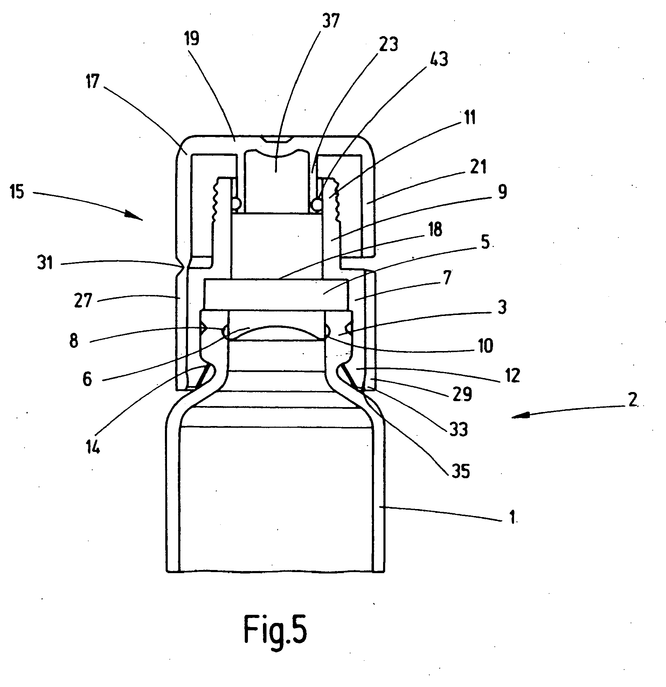

Figur 5 zeigt eine weitere Ausführungsform der Spritze 2. Gleiche Teile sind mit gleichen Bezugsziffern versehen, sodass auf eine erneute Beschreibung der Teile verzichtet wird. In diesem Fall kann der Zapfen 23 des ersten Teilbereichs 17 der zweiteiligen Kappe 15 kürzer ausgebildet sein. Er greift in das Innere des Ansatzes 9. Der Außendurchmesser des Zapfens 23 kann so auf den Innendruchmesser des Ansatzes 9 abgestimmt sein, dass ein radialer Dichtbereich geschaffen wird. Es kann auch mindestens eine radial von der Außenfläche des Zapfens 23 der Kappe 15 vorstehende ringförmige Wulst vorgesehen werden, die gegen die Innenfläche des Ansatzes 9 drückt. Eine Wulst kann aber auch radial von der Innenfläche des Ansatzes 9 des Verschlusselements 7 hervorstehen und gegen die Außenfläche des Zapfens 23 drücken, um einen Dichtbereich zu realisieren.

Eine weitere Möglichkeit besteht darin, dass der Dichtbereich durch mindestens ein separates - vorzugsweise als O-Ring 43 ausgebildetes - Dichtelement realisiert wird. Dieses liegt dann zwischen der Innenfläche des Ansatzes 9 und der Außenfläche des Zapfens 23. Zur Lagefixierung des Dichtelements kann auf der Außenfläche des Zapfens 23 und/oder der Innenfläche des Ansatzes 9 eine Ringnut vorgesehen werden.

Nach allem zeigt sich, dass auf einfache Weise der Einstichbereich 18 der Membran 5 steril abgedeckt werden kann, ohne dass es dazu irgendwelcher axialer Kräfte bedürfte, die von einer Kappe 15 aufzubringen wären. Eine Sollbruchlinie 31 der Kappe 15 bleibt auf diese Weist unbelastet und kann nicht ungewollt aufreißen.

Die Abdeckeinrichtung der Spritze 2 oder Karpule wird dabei entweder durch eine Siegelfolie 13 realisiert, die am Ende 3 des Zylinders 1 mittels einer Klebeverbindung befestigt werden kann. Die Klebeverbindung kann durch üblichen Klebstoff oder aber durch Ultraschall-Schweißverfahren, Hitze oder dergleichen realisiert werden. Die Siegelfolie 13 überspannt den Einstichbereich 18 der Membran 5 und schützt diesen sicher vor Verunreinigungen.

Die Abdeckeinrichtung und deren Dichtbereich kann auch dadurch realisiert werden, dass an einer Kappe 15 und/oder der Membran 5 ein Vorsprung und entsprechend an der Membran 5 und/oder der Kappe 15 eine Ausnehmung vorgesehen wird. Der Vorsprung weist eine Außenfläche auf, deren Außendurchmesser so auf den Innendurchmesser der Ausnehmung abgestimmt ist, das im Berührungsbereich eine radiale Dichtung realisiert wird. Axiale Kräfte zur Realisierung der Dichtung sind also nicht erforderlich.

Die Dichtwirkung kann durch mindesten einen ringförmigen Vorsprung, also eine Wulst, auf der Außenseite des Vorsprungs und/oder der Innenseite der Ausnehmung verbessert werden. Auch können separate Dichtelemente, beispielsweise ein O-Ring 43 im Berührungsbereich zwischen der Außenfläche des Vorsprungs und der Innenfläche der Ausnehmung vorgesehen werden, um die radiale Dichtung zu realisieren.

Betrachtet man das Ausführungsbeispiel gemäß Figur 5, so zeigt sich, dass die in Richtung der gedachten Mittelachse gemessene Länge des Vorsprungs 23 und die des Ansatzes 29 so aufeinander abgestimmt ist, dass sich ein radialer Dichtbereich ergibt, entweder dadurch, dass die Außenfläche des Vorsprungs 23 an der Innenfläche des Ansatzes 9 anliegt, oder dadurch, dass eine Wulst entweder auf der Außenfläche des Vorsprungs 23 und/oder der Innenfläche des Ansatzes 9 vorgesehen ist. Schließlich kann auch der oben angesprochene O-Ring 43 vorgesehen werden, um die radiale Dichtung zu gewährleisten. Aus Figur 5 wird noch deutlich, dass der Vorsprung 23 in den Fällen, in denen er von dem Ansatz 9 umgriffen wird, nicht hohl ausgebildet zu sein braucht.

Insgesamt zeigt sich, dass die Abdeckeinrichtung und der Dichtbereich einfach und kostengünstig realisierbar sind, und dass die Sollbruchlinie 31 der Kappe 15 nicht mehr durch axiale Kräfte beaufschlagt wird, die ein ungewollte Aufreisen der Sollbruchlinie, also abreisen von Abreisstegen 32 bewirken könnten.

## Patentansprüche

1. Spritze (2) oder Karpule mit
einem Zylinder (1), der einen Hohlraum umschließt und ein Ende (3) umfasst, das einen Zugang zum Hohlraum aufweist,
- einer Membran (5), die den Zugang dicht abschließt und einen von einer Nadel durchstechbaren Einstichbereich (18) aufweist,
- einer eine Sollbruchlinie (31) aufweisenden, die Membran überspannenden zweiteiligen Kappe (15), und mit
- einer Abdeckeinrichtung, die den Einstichbereich (18) der Membran (5) steril abdeckt und die einen den Einstichbereich (18) überspannenden Dichtbereich umfasst,
**dadurch gekennzeichnet, dass**
- der Dichtbereich unabhängig von im Bereich der Sollbruchlinie (31) aufzubringenden Axialkräften den Einstichbereich (18) steril abdeckt.

2. Spritze nach Anspruch 1, **dadurch gekennzeichnet, dass** der Dichtbereich eine den Einstichbereich (18) überspannende Siegelfolie (13) umfasst.

3. Spritze nach Anspruch 2, **dadurch gekennzeichnet, dass** die Siegelfolie (13) an dem Ende (3) des Zylinders (1) mittels einer Klebeverbindung befestigt ist.

4. Spritze nach Anspruch 2 oder 3, **dadurch gekennzeichnet, dass** die Siegelfolie (13) Kunststoff und/oder Aluminium umfasst.

5. Spritze nach einem der Ansprüche 2 bis 4, **dadurch gekennzeichnet, dass** die Siegelfolie (13) mindestens eine Abziehlasche (16) aufweist.

6. Spritze nach Anspruch 1, **dadurch gekennzeichnet, dass** die Abdeckeinrichtung einen Vorsprung und eine den Vorsprung zumindest bereichsweise aufnehmende Ausnehmung aufweist, und dass der Außendurchmesser des Vorsprungs so auf den Innendurchmesser der Ausnehmung abgestimmt ist, dass im Berührungsbereich ein Dichtbereich mit radialer Dichtung realisiert wird.

7. Spritze nach Anspruch 6, **dadurch gekennzeichnet, dass** die Kappe (15), auf ihrer der Membran (5) zugewandten Innenseite, einen Zapfen (23) aufweist.

8. Spritze nach Anspruch 7, **dadurch gekennzeichnet, dass** der Zapfen (23) den Vorsprung bildet.

9. Spritze nach einem der Ansprüche 6 bis 8, **dadurch gekennzeichnet, dass** die Membran (5) die Ausnehmung (24) aufweist.

10. Spritze nach einem der Ansprüche 6 bis 9, **dadurch gekennzeichnet, dass** der Dichtbereich mindestens einen radial von der Innenfläche der Ausnehmung der Membran (5) und/oder von der Außenfläche des Vorsprungs (23) hervorstehende Wulst aufweist.

11. Spritze nach Anspruch 7, **dadurch gekennzeichnet, dass** der Zapfen (23) die Ausnehmung (37) aufweist und dass die Membran (5) den Vorsprung (33) aufweist.

12. Spritze nach Anspruch 10 oder 11, **dadurch gekennzeichnet, dass** der Dichtbereich durch mindestens eine radial an der Außenfläche des Vorsprungs (39) der Membran (5) und/oder der Innenfläche der Ausnehmung (37) vorstehende Wulst realisiert ist.

13. Spritze nach Anspruch 7, **dadurch gekennzeichnet, dass** am Ende (3) des Zylinders (1) ein Verschlusselement (7) mit einem hohl ausgebildeten Ansatz (9) vorgesehen ist.

14. Spritze nach Anspruch 13, **dadurch gekennzeichnet, dass** das Innere des Ansatzes (9) die Ausnehmung darstellt, in die der Vorsprung (23) der Kappe eingreift.

15. Spritze nach Anspruch 13 oder 14, **dadurch gekennzeichnet, dass** der Dichtbereich mindestens eine radial von der Außenfläche des Vorsprungs (23) der Kappe (15) vorstehende Wulst aufweist.

16. Spritze nach einem der Ansprüche 13 bis 15, **dadurch gekennzeichnet, dass** der Dichtbereich mindestens eine radial von der Innenfläche des Ansatzes (9) hervorstehende Wulst aufweist.

17. Spritze nach einem der Ansprüche 13 bis 16, **dadurch gekennzeichnet, dass** der Dichtbereich mindesten ein separates - vorzugsweise als O-Ring (43) ausgebildetes - Dichtelement aufweist.

## Claims

1. Syringe (2) or carpule with
- a cylinder (1) that surrounds a cavity and comprises an end (3) that has an access to the cavity,
- a membrane (5) that seals the access and a puncture area (18) that can be punctured by a needle,
- a two-part cap (15) having a predetermined breaking line (31) and spanning the membrane and with
- a cover device that covers the puncture area (18) of the membrane (5) in a sterile manner and that comprises a sealing area spanning the puncture area (18),
**characterized in that**
- the sealing area covers the puncture area (18) in a sterile manner regardless of axial forces to be applied in the area of the predetermined breaking line (31).

2. Syringe according to claim 1, **characterized in that** the sealing area comprises a sealing foil (13) spanning the puncture area (18).

3. Syringe according to claim 2, **characterized in that** the sealing foil (13) is attached at the end (3) of the cylinder (1) by means of an adhesive connection.

4. Syringe according to claim 2 or 3, **characterized in that** the sealing foil (13) comprises plastic and/or aluminum.

5. Syringe according to one of claims 2 through 4, **characterized in that** the sealing foil (13) has at least one pull-off tab (16).

6. Syringe according to claim 1, **characterized in that** the cover device has a projection and a recess accommodating the projection at least in some parts, and that the outer diameter of the projection is coordinated with the inner diameter of the recess such that a sealing area with a radial seal is realized in the contact area.

7. Syringe according to claim 6, **characterized in that** the cap (15) has a neck (23) on the inside facing towards the membrane (5).

8. Syringe according to claim 7, **characterized in that** the neck (23) forms the projection.

9. Syringe according to one of claims 6 through 8, **characterized in that** the membrane (5) comprises the recess (24).

10. Syringe according to one of claims 6 through 9, **characterized in that** the sealing area has at least one bead projection radially from the inner surface of the recess of the membrane (5) and/or from the outer surface of the projection (23).

11. Syringe according to claim 7, **characterized in that** the neck (23) has the recess (37) and that the membrane (5) has the projection (33).

12. Syringe according to claim 10 or 11, **characterized in that** the sealing area is realized by at least one bead projecting radially on the outer area of the projection (39) of the membrane (5) and/or the inner area of the recess (37).

13. Syringe according to claim 7, **characterized in that** a closure element (7) with a lug (3) embodied in a hollow manner is provided on the end (3) of the cylinder (1).

14. Syringe according to claim 13, **characterized in that** the interior of the lug (9) represents the recess in which the projection (23) of the cap engages.

15. Syringe according to claim 13 or 14, **characterized in that** the sealing area has at least one bead projecting radially from the outer surface of the projection (23) of the cap (15).

16. Syringe according to one of claims 13 through 15, **characterized in that** the sealing area has at least one bead projecting radially from the inner surface of the lug (9).

17. Syringe according to one of claims 13 through 16, **characterized in that** the sealing area has at least one separate sealing element, preferably embodied as an O-ring (43).

## Revendications

1. Seringue (2) ou carpule comprenant
- un cylindre (1) qui entoure un espace creux et comprend une extrémité (3) qui présente un accès à l'espace creux,
- une membrane (5) qui ferme l'accès de manière étanche et présente une zone de piqûre (18) pouvant être transpercée par une aiguille,
- un capuchon (15) en deux parties présentant une ligne de rupture de consigne (31), recouvrant la membrane, et comprenant
- un dispositif de recouvrement qui recouvre de manière stérile la zone de piqûre (18) de la membrane (5) et comprend une zone étanche recouvrant la zone de piqûre (18),
**caractérisée en ce que**
- - la zone étanche recouvre de manière stérile la zone de piqûre (18), indépendamment de forces axiales devant être introduites dans la région de la ligne de rupture de consigne (31).

2. Seringue selon la revendication 1, **caractérisée en ce que** la zone étanche comprend un film de scellement (13) recouvrant la zone de piqûre (18).

3. Seringue selon la revendication 2, **caractérisée en ce que** le film de scellement (13) est fixé à l'extrémité (3) du cylindre (1) au moyen d'une connexion adhésive.

4. Seringue selon la revendication 2 ou 3, **caractérisée en ce que** le film de scellement (13) comprend du plastique et/ou de l'aluminium.

5. Seringue selon l'une quelconque des revendications 2 à 4, **caractérisée en ce que** le film de scellement (13) présente au moins une languette à tirer (16).

6. Seringue selon la revendication 1, **caractérisée en ce que** le dispositif de recouvrement présente une saillie et un évidement recevant la saillie au moins par région, et que le diamètre externe de la saillie est adapté au diamètre interne de l'évidement, de sorte qu'une zone étanche est réalisée avec un joint radial dans la région de contact.

7. Seringue selon la revendication 6, **caractérisée en ce que** le capuchon (15) présente un tourillon (23) sur son côté interne tourné vers la membrane (5).

8. Seringue selon la revendication 7, **caractérisée en ce que** le tourillon (23) forme la saillie.

9. Seringue selon l'une quelconque des revendications 6 à 8, **caractérisée en ce que** la membrane (5) présente l'évidement (24).

10. Seringue selon l'une quelconque des revendications 6 à 9, **caractérisée en ce que** la zone étanche présente au moins un bourrelet dépassant radialement de la surface interne de l'évidement de la membrane (5) et/ou de la surface externe de la saillie (23).

11. Seringue selon la revendication 7, **caractérisée en ce que** le tourillon (23) présente l'évidement (37) et que la membrane (5) présente la saillie (33).

12. Seringue selon la revendication 10 ou 11, **caractérisée en ce que** la zone étanche est réalisée par au moins un bourrelet faisant saillie radialement sur la surface externe de la saillie (39) de la membrane (5) et/ou la surface interne de l'évidement (37).

13. Seringue selon la revendication 7, **caractérisée en ce qu'**un élément de fermeture (7) avec un épaulement (9) réalisé de manière creuse est prévu à l'extrémité (3) du cylindre (1).

14. Seringue selon la revendication 13, **caractérisée en ce que** l'intérieur de l'épaulement (9) représente l'évidement, dans lequel la saillie (23) du capuchon s'engrène.

15. Seringue selon la revendication 13 ou 14, **caractérisée en ce que** la zone étanche présente au moins un bourrelet faisant saillie radialement de la surface externe de la saillie (23) du capuchon (15).

16. Seringue selon l'une quelconque des revendications 13 à 15, **caractérisée en ce que** la zone étanche présente au moins un bourrelet dépassant radialement de la surface interne de l'épaulement (9).

17. Seringue selon l'une quelconque des revendications 13 à 16, **caractérisée en ce que** la zone étanche présente au moins un élément d'étanchéité séparé, réalisé de préférence en tant que joint torique (43).
